(19) Europäisches Patentamt / European Patent Office / Office européen des brevets

(11) EP 4 458 803 A1

(12) # EUROPEAN PATENT APPLICATION
published in accordance with Art. 153(4) EPC

(43) Date of publication:
**06.11.2024 Bulletin 2024/45**

(21) Application number: **23895026.5**

(22) Date of filing: **22.11.2023**

(51) International Patent Classification (IPC):
***C07C 69/86*** *(2006.01)* ***C07C 67/60*** *(2006.01)*
***C07C 67/02*** *(2006.01)* ***C07C 67/03*** *(2006.01)*
***C08J 11/10*** *(2006.01)* ***C08G 63/60*** *(2006.01)*

(52) Cooperative Patent Classification (CPC):
**C07C 67/02; C07C 67/03; C07C 67/60; C07C 69/86; C08G 63/60; C08J 11/10;** Y02W 30/62

(86) International application number:
**PCT/KR2023/018887**

(87) International publication number:
**WO 2024/112099 (30.05.2024 Gazette 2024/22)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.11.2022 KR 20220158778**

(71) Applicant: **SK Chemicals Co., Ltd.**
**Seongnam-si, Gyeonggi-do 13494 (KR)**

(72) Inventors:
- **YOO, Young-Man**
  **Seongnam-si, Gyeonggi-do 13494 (KR)**
- **HWANG, Eun-Yeong**
  **Seongnam-si, Gyeonggi-do 13494 (KR)**

(74) Representative: **Dehns**
**10 Old Bailey**
**London EC4M 7NG (GB)**

(54) **RECYCLED BIS(4-HYDROXYBUTYL)TEREPHTHALATE, METHOD FOR PREPARING SAME, AND POLYESTER RESIN USING SAME**

(57) A recycled bis(4-hydroxybutyl)terephthalate according to an embodiment has a low residual amount of ethylene glycol derivatives, and thus, can be used as a polymerization raw material for high-crystallinity engineering polyester products or biodegradable polyester, and provides excellent quality of, for example, color.

[Fig. 1]

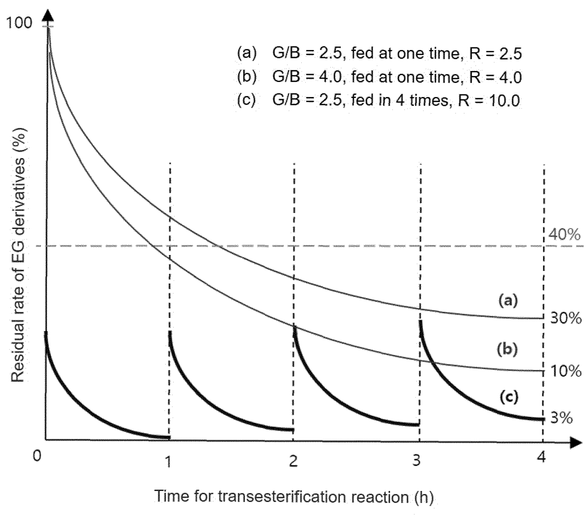




Processed by Luminess, 75001 PARIS (FR)

## Description

### Technical Field

The present invention relates to the preparation of a bis(4-hydroxybutyl) terephthalate with high purity, as a raw material for a polyester resin, using recycled monomers and to polyester resins and articles obtained therefrom.

### Background Art

Polyester is widely used as a material for beverage-filling containers, packaging films, audio and video films, and the like by virtue of its excellent mechanical strength, thermal resistance, transparency, and gas barrier properties. In addition, polyester is widely produced worldwide as an industrial material such as medical fibers and tire cords. In particular, polyester sheets or plates have good transparency and excellent mechanical strength, so that they are widely used as raw materials for cases, boxes, partitions, shelves, panels, packaging materials, building materials, interior and exterior materials, and the like.

As a result, waste of plastics such as polyester is generated globally at an unmanageable level every year. Recently, countries around the world have prepared regulations and plans for recycling waste plastic resources, including waste polyester. Although physical or chemical methods are used as methods of recycling waste polyester, physical recycling methods cannot guarantee purity and, therefore, are not widely used.

In chemical recycling methods, the ester bond of waste polyester is cleaved to depolymerize it. Reactions such as glycolysis, hydrolysis, methanolysis, and aminolysis are used. Glycolysis among them is to decompose waste polyester by adding a glycol such as ethylene glycol or diethylene glycol at high temperatures. For example, bis(2-hydroxyethyl) terephthalate (BHET) can be obtained through the glycolysis of waste polyethylene terephthalate (PET), and a technique of using it as a raw material for preparing a polyester resin is known.

However, from the viewpoint of environmental friendliness, it is more meaningful to convert waste PET products, which generally have a short lifespan, into engineering polyester products or environmentally friendly biodegradable polyester products that have a long lifespan. In this regard, recently, attempts have been made to regenerate polyester resins other than PET through the transesterification of waste PET and a glycol. Since an excessive amount of a glycol is used in this reaction and the reaction time is long, there is a problem in that a large amount of by-products such as cyclic ester compounds are formed as a side reaction of the glycol.

[Prior Art Document]

(Non-patent Document 1) Park, S.H., Kim, S.H., Polyethylene terephthalate) recycling for high value added textiles, Fashion and Textiles 1, 1 (2014)

### Disclosure of Invention

### Technical Problem

The present inventors have attempted to develop a method capable of minimizing the formation of by-products without using an excessive amount of a glycol through a technology to recycle BHET obtained by the depolymerization of waste PET-based products into various engineering polyester products or environmentally friendly biodegradable polyester products.

Meanwhile, recycled BHET has generally low purity due to impurities formed from the reagents used in the depolymerization process and side reactions, so that high-cost processes such as ion exchange or recrystallization are required to purify it, making it difficult to be commercially utilized.

As a result of research conducted to solve this problem, it has been possible to obtain recycled bis(4-hydroxybutyl) terephthalate with high purity, while using BHET with low purity or reducing the amount of 1,4-butanediol used, by introducing BHET in a divided or continuous manner into a transesterification reaction with 1,4-butanediol. It has been possible to use the same as a raw material for preparing engineering polyester products with high quality or environmentally friendly biodegradable polyester products.

Accordingly, an object of the present invention is to prepare recycled bis(4-hydroxybutyl) terephthalate with high purity by using recycled BHET without using an excessive amount of 1,4-butanediol, to provide raw materials for the polymerization of various polyester resins with high quality, and to provide various polyester resins and articles with high quality therefrom.

## Solution to Problem

According to an aspect of the present invention, there is provided recycled bis(4-hydroxybutyl) terephthalate in which the total content of ethylene glycol, diethylene glycol, and their derivatives, as measured by $^1$H-NMR, is 20% by mole or less based on the total number of moles of the entire glycols and their derivatives, and, when it is prepared into a specimen having a thickness of 6 mm, the color-b value in the Hunter Lab color space is 8 or less.

According to another aspect of the present invention, there is provided a process for preparing recycled bis(4-hydroxybutyl) terephthalate, which comprises (1) feeding 1,4-butanediol to a reactor; and (2) feeding bis(2-hydroxyethyl) terephthalate to the reactor in a divided or continuous manner and carrying out a transesterification reaction, wherein the following Relationship (1) is satisfied:

$$1.5 \leq G/B \leq 3.5 \ldots (1)$$

In Relationship (1), G is the total number of moles of 1,4-butanediol fed to the reactor, and B is the total number of moles of bis(2-hydroxyethyl) terephthalate fed to the reactor.

According to another aspect of the present invention, there is provided a raw material for the polymerization of a polyester resin, which comprises recycled bis(4-hydroxybutyl) terephthalate.

According to another aspect of the present invention, there is provided a process for preparing a polyester resin, which comprises (1) feeding 1,4-butanediol to a reactor; (2) feeding bis(2-hydroxyethyl) terephthalate to the reactor in a divided or continuous manner and carrying out a transesterification reaction; and (3) subjecting the product of the transesterification reaction to a polycondensation reaction, wherein the above Relationship (1) is satisfied:
According to another aspect of the present invention, there is provided a polyester resin prepared by the above process.

## Advantageous Effects of Invention

Since the recycled bis(4-hydroxybutyl) terephthalate according to the present invention has a low content of ethylene glycol derivatives, a polyester resin having excellent crystallinity and purity can be provided. In addition, since the recycled bis(4-hydroxybutyl) terephthalate according to the present invention has excellent quality, such as color, and is easily crushed to produce flakes or powder, it is suitable for use as a raw material for various polymers.

Specifically, according to the present invention, it is possible to prepare a 1,4-butanediol-derived polybutylene terephthalate-based polyester resin (PBT, TPEE, or the like) with high purity. It is possible to produce high value-added engineering polyester products or biodegradable polyester (PBAT or the like) products with low ethylene glycol components that affect ecotoxicity from waste PET products.

In particular, according to the present invention, in the preparation of bis(4-hydroxybutyl) terephthalate through a transesterification reaction of BHET and 1,4-butanediol, a constant amount of BHET relative to 1,4-butanediol is introduced in a divided or continuous manner into the reaction with 1,4-butanediol, whereby it is possible to increase purity while BHET with low purity is used, or the amount of 1,4-butanediol is reduced, resulting in an advantage in terms of costs.

## Brief Description of Drawings

Fig. 1 shows the change in the residual rate of ethylene glycol derivatives over time with respect to the feeding method during a transesterification reaction.

## Best Mode for Carrying out the Invention

Hereinafter, the present invention will be described in more detail.

In this specification, terms referring to the respective components are used to distinguish them from each other and are not intended to limit the scope of the embodiment. In addition, in the present specification, a singular expression is interpreted to cover a plural number as well unless otherwise specified in the context.

In the present specification, the terms first, second, and the like are used to describe various components. But the components should not be limited by the terms. The terms are used for the purpose of distinguishing one element from another.

In the present specification, the term "comprising" is intended to specify a particular characteristic, region, step, process, element, and/or component. It does not exclude the presence or addition of any other characteristic, region, step, process, element and/or component, unless specifically stated to the contrary.

The molecular weight of a compound or polymer described in the present specification, for example, a number

average molecular weight or a weight average molecular weight, is a relative mass based on carbon-12 as is well known. Although its unit is not described, it may be understood as a molar mass (g/mole) of the same numerical value, if necessary.

In the present specification, a "derivative" of a specific compound refers to a compound obtained by partially transforming the compound through a chemical reaction or combining it with other components, thereby containing the main part of the compound.

In the present specification, a unit or group "derived" from a specific component refers to a part of the component contained in a final product through a chemical reaction such as a polymerization reaction. It may be present in a form in which a part thereof is modified during the reaction or it is combined with other components. For example, a unit or group derived from at least one monomer is contained in the chain constituting a polymer.

According to an aspect of the present invention, there is provided a process for preparing recycled bis(4-hydroxybutyl) terephthalate, which can be used as a raw material for various polymers, through a transesterification reaction of bis(2-hydroxyethyl) terephthalate and 1,4-butanediol.

The process for preparing recycled bis(4-hydroxybutyl) terephthalate comprises (1) feeding 1,4-butanediol to a reactor; and (2) feeding bis(2-hydroxyethyl) terephthalate to the reactor in a divided or continuous manner and carrying out a transesterification reaction.

**Bis(2-hydroxyethyl) terephthalate**

In bis(2-hydroxyethyl) terephthalate used in the present invention, an ethylene glycol residue is substituted with a 1,4-butanediol residue through a transesterification reaction. Thereafter, it may form the polymer chain of a final polyester resin through a polymerization reaction. Bis(2-hydroxyethyl) terephthalate is an ester of two ethylene glycols and one terephthalic acid. For example, it is a compound formed as an intermediate in the process of preparing a polyester such as polyethylene terephthalate (PET) through the polymerization of ethylene glycol and terephthalic acid or its ester.

The bis(2-hydroxyethyl) terephthalate used in the present invention may be obtained by the depolymerization of waste polyester. For example, the bis(2-hydroxyethyl) terephthalate may be obtained from waste polyester having a repeat unit of ethylene glycol and terephthalic acid like polyethylene terephthalate (PET) or glycol-modified polyethylene terephthalate (PETG). Specifically, it may be obtained by well-known depolymerization methods such as glycolysis, hydrolysis, and methanolysis. In particular, the bis(2-hydroxyethyl) terephthalate may be obtained by depolymerizing waste polyethylene terephthalate with ethylene glycol and then purifying it.

Since the recycled bis(2-hydroxyethyl) terephthalate is subjected to several chemical steps in the process of preparing it from waste plastic, bis(2-hydroxyethyl) terephthalate having various purities is commercially available depending on the type of waste plastic and the depolymerization and purification methods.

Recycled bis(2-hydroxyethyl) terephthalate obtained by the depolymerization of waste polyester as described above (referred to as recycled BHET or abbreviated as rBHET or rBHET) may contain reagents or solvents used in various chemical steps during the depolymerization of waste polyester, or by-products formed by side reactions with them. Thus, recycled bis(2-hydroxyethyl) terephthalate obtained by the depolymerization of waste polyester as described above needs to be understood as distinct from a pure BHET compound. For this reason, recycled BHET can also be viewed as a kind of composition comprising two or more components, i.e., a BHET composition. Such recycled BHET may be used as a raw material for the polymerization of a polyester resin. BHET recycled by a common depolymerization process contains organic and inorganic impurities in addition to BHET as the main component; thus, its purity is not high.

Impurities contained in the recycled BHET may comprise, for example, diethylene glycol derivatives and unreacted monomers. The total content of impurities contained in the recycled BHET may be 1% by weight or more, 3% by weight or more, or 5% by weight or more, and may be 30% by weight or less, 25% by weight or less, 20% by weight or less, or 15% by weight or less.

The purity of the recycled BHET may be measured using liquid chromatography or the like. Specifically, the purity of the recycled BHET may be calculated by measuring the fraction (%) of the peak area of BHET out of the total peak area in a spectrum obtained using high-performance liquid chromatography (HPLC).

For example, the purity of the recycled BHET may be 99% or less, 97% or less, or 95% or less, and may be 70% or more, 75% or more, or 80% or more. Specifically, the purity of the BHET introduced into the transesterification reaction may be 70% to 99%, more specifically, 75% to 97% or 80% to 95%.

**Transesterification reaction**

1,4-Butanediol and bis(2-hydroxyethyl) terephthalate are subjected to a transesterification reaction.

[Reaction Scheme 1]

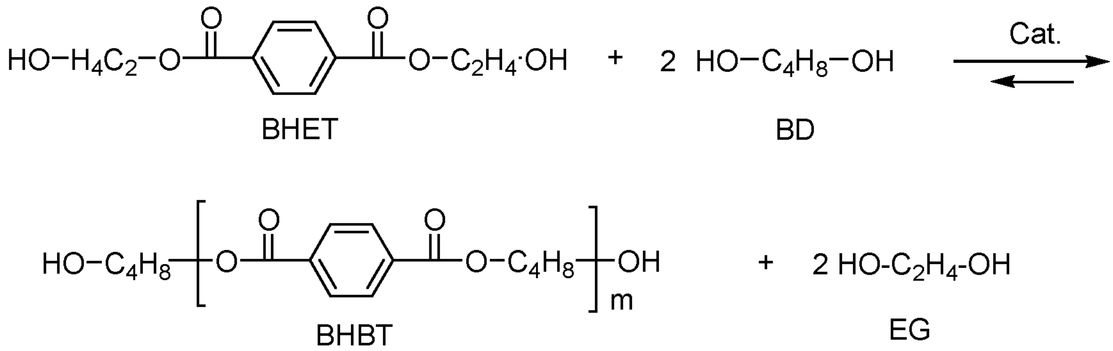


BHET BD

BHBT EG

1,4-Butanediol forms a residue of the reaction product, bis(4-hydroxybutyl) terephthalate, or an oligomer thereof, through the transesterification reaction. The bis(4-hydroxybutyl) terephthalate or an oligomer thereof may form the polymer chain of a final polyester resin.

1,4-Butanediol introduced into the transesterification reaction has a boiling point higher than that of ethylene glycol by 30°C or more, which is advantageous for purification through fractional distillation during the reaction.

The transesterification reaction may be carried out in the presence of a catalyst. Thus, when 1,4-butanediol or bis(2-hydroxyethyl) terephthalate is fed to a reactor, a catalyst may be fed together.

As the catalyst for the transesterification reaction, for example, at least one selected from the group consisting of a titanium-based catalyst, a germanium-based catalyst, an antimony-based catalyst, an aluminum-based catalyst, and a tin-based catalyst may be used.

Examples of the titanium-based catalyst include tetraethyl titanate, acetyltripropyl titanate, tetrapropyl titanate, tetrabutyl titanate, 2-ethylhexyl titanate, octylene glycol titanate, triethanolamine titanate, acetylacetonate titanate, ethylacetoacetic ester titanate, isostearyl titanate, titanium dioxide, and the like. Examples of the germanium-based catalyst include germanium dioxide, germanium tetrachloride, germanium ethylene glycol oxide, germanium acetate, coprecipitates thereof, or mixtures thereof. Specifically, germanium dioxide can be used as the germanium-based catalyst. Both crystalline and amorphous germanium dioxide may be used as the germanium dioxide, and glycol-soluble ones may also be used.

The amount of the transesterification catalyst employed may vary depending on the reaction conditions and the catalyst used. As an example, a metal-based catalyst (e.g., titanium-based catalyst and tin-based catalyst) may be employed such that the weight of the metal-based catalyst is 10 ppm to 300 ppm based on the total weight of bis(2-hydroxyethyl) terephthalate fed to the reactor. If it is less than 10 ppm, the transesterification reaction rate is too slow; thus, it may be difficult to obtain the desired recycled bis(4-hydroxybutyl) terephthalate with high purity. If it exceeds 300 ppm, the color-b value of the recycled bis(4-hydroxybutyl) terephthalate obtained may be too high; thus, it may not be suitable as a raw material for the polymerization of a polyester resin.

The transesterification reaction may be carried out in a batch or continuous type.

As an example, when 1,4-butanediol is fed to a reactor, and the temperature reaches a certain level as the temperature is raised, bis(2-hydroxyethyl) terephthalate may be fed.

In addition, the transesterification reaction may be carried out under a nitrogen atmosphere.

For example, the feeding of bis(2-hydroxyethyl) terephthalate may be carried out in a nitrogen atmosphere at a temperature of 165°C to 220°C while ethylene glycol as a by-product is removed.

According to the present invention, bis(2-hydroxyethyl) terephthalate is introduced into the transesterification reaction with 1,4-butanediol in a divided or continuous manner.

According to an embodiment, bis(2-hydroxyethyl) terephthalate is introduced into the transesterification reaction with 1,4-butanediol in a divided manner of two or more times.

The number of divided introductions may be 2 times or more, 3 times or more, 4 times or more, or 5 times or more, and may be 100 times or fewer, 50 times or fewer, 30 times or fewer, 20 times or fewer, 15 times or fewer, or 10 times or fewer. As a specific example, the number of divided introductions may be 2 to 30 times or 3 to 15 times.

The time interval between the divided introductions may be determined by dividing the total introduction time period by the number of divided introductions. The total introduction time may be, for example, 1 hour or longer or 2 hours or longer, and may be 5 hours or shorter or 4 hours or shorter.

In addition, the amount of introduction at one time during the divided introductions may be determined by dividing the total amount of bis(2-hydroxyethyl) terephthalate to be introduced into the reaction by the number of divided introductions.

According to the present invention, the total amount of the components introduced into the transesterification

reaction satisfies the following Relationship (1).

$$1.5 \leq G/B \leq 3.5 \ ...\ (1)$$

In Relationship (1), G is the total number of moles of 1,4-butanediol fed to the reactor, and B is the total number of moles of bis(2-hydroxyethyl) terephthalate fed to the reactor.
If G/B is less than 1.5, there are many ethylene glycol residues derived from BHET, which may deteriorate the purity of recycled bis(4-hydroxybutyl) terephthalate. If G/B exceeds 3.5, an excessive amount of 1,4-butanediol is fed, which is not preferable. For example, G/B may be 1.5 or more, 1.8 or more, or 2.0 or more, and may be 3.5 or less, 3.0 or less, or 2.5 or less. As a specific example, G/B may be 1.5 to 3.0, or 1.5 to 2.5.
The number of divided introductions and the total introduction amount may be more effectively controlled by the following Relationship (2). As an example, the introduction ratio (R) of bis(2-hydroxyethyl) terephthalate according to Relationship (2) may be 5 or more.

$$R = G \times \frac{N}{B} \ ...\ (2)$$

In Relationship (2), G is the total number of moles of 1,4-butanediol fed to the reactor, B is the total number of moles of bis(2-hydroxyethyl) terephthalate fed to the reactor, and N is the number of divided introductions of the bis(2-hydroxyethyl) terephthalate.
For example, the R value may be 5 or more, 6 or more, 8 or more, or 10 or more, and may be 100 or less, 50 or less, 30 or less, or 20 or less. As a specific example, it may be 4 to 50 or 6 to 30.
Fig. 1 shows the change in the residual rate of ethylene glycol derivatives over time with respect to the feeding method during a transesterification reaction.
Referring to Fig. 1, (a) if it is introduced at one time with a G/B set to 2.5 (i.e., R = 2.5), the amount of ethylene glycol derivatives (mainly BHET) decreases as the reaction progresses from the maximum (100%) at the beginning of the introduction; however, even at the time of completion of the reaction, the residual rate of ethylene glycol derivatives remains at a high level (30%); (b) if it is introduced at one time with a G/B set to 4.0 (i.e., R = 4.0), the amount of ethylene glycol derivative decreases more steeply and reaches a low level (10%) at the time of completion of the reaction; and (c) if it is introduced as divided in 4 times with a G/B set to 2.5 (i.e., R = 10.0), the amount of ethylene glycol derivative is maintained at a certain level (40%) or less throughout the entire reaction process, and, especially, it reaches a very low level (3%) at the time of completion of the reaction.
In (a) and (b) of Fig. 1, as conventional methods, an excessive amount of 1,4-butanediol is required to lower the residual rate of the final ethylene glycol derivatives, which increases the cost. In contrast, in (c), as a method of the present invention, the residual amount of ethylene glycol derivatives in the final product may be at a very low level even without increasing the amount of 1,4-butanediol.
According to another embodiment, bis(2-hydroxyethyl) terephthalate is introduced into the transesterification reaction with 1,4-butanediol in a continuous manner.
The total time of continuous introduction may be, for example, 1 hour or longer or 2 hours or longer, and may be 5 hours or shorter or 4 hours or shorter.
For example, the continuous introduction may be to introduce a constant amount of bis(2-hydroxyethyl) terephthalate per hour. The amount of introduction per hour may be determined by dividing the total amount of bis(2-hydroxyethyl) terephthalate to be introduced into the reaction by the total introduction time.
Specifically, bis(2-hydroxyethyl) terephthalate may be dissolved in water at 80 to 100°C to prepare an aqueous BHET solution having a concentration of about 10 to 20% by weight, which may be continuously introduced into a transesterification reaction. The continuous introduction may be carried out, for example, up to 1 hour before the termination of the reaction. A dropping funnel may be used at the laboratory level, or a metered feeder may be used at the commercial level, for the continuous introduction of a constant amount.
As a more specific example, a total amount of 577 g of bis(2-hydroxyethyl) terephthalate to be introduced is dissolved in 3,861 g of purified water at 90°C to prepare a slurry, and the aqueous BHET solution may be metered at 18.5 g per minute for 4 hours out of the total 5 hours required for the transesterification reaction.
When the introduction of bis(2-hydroxyethyl) terephthalate in a divided or continuous manner is completed, the reaction conditions may be maintained until the transesterification reaction is completed. In addition, a step of removing ethylene glycol as a by-product in a nitrogen atmosphere may be continued during the transesterification reaction. The removal of ethylene glycol may be carried out by a distillation process using a difference in boiling point from other

components. For effective removal by distillation, nitrogen, an inert gas, may be flowed. Ethylene glycol thus distilled off may be recovered by cooling and reused in other processes.

The termination point of the transesterification reaction may be determined by considering the theoretical amount of ethylene glycol formed from bis(2-hydroxyethyl) terephthalate through the transesterification reaction or as the point when the by-product is no longer released.

The pressure in the reactor for the transesterification reaction in a nitrogen atmosphere may be, for example, 0.01 $kg/cm^2$ or more, 0.05 $kg/cm^2$ or more, or 0.1 $kg/cm^2$ or more, and may be 0.5 $kg/cm^2$ or less, 0.3 $kg/cm^2$ or less, 0.2 $kg/cm^2$ or less, or 1.5 $kg/cm^2$ or less. The temperature during the transesterification reaction may be 165°C or higher, 170°C or higher, 180°C or higher, or 190°C or higher, and may be 225°C or lower, 220°C or lower, 215°C or lower, 210°C or lower, or 200°C or lower. Specifically, the transesterification reaction may be carried out at a temperature of 170°C or higher to smoothly remove ethylene glycol as a by-product. In addition, it may be carried out at a temperature lower than the boiling point of 1,4-butanediol by 10°C in order to reduce the loss of 1,4-butanediol for the substitution. As a more specific example, the temperature during the transesterification reaction with 1,4-butanediol may be adjusted to 170°C to 220°C.

**Recycled bis(4-hydroxybutyl) terephthalate**

The product of the transesterification reaction mainly comprises bis(4-hydroxybutyl) terephthalate and derivatives thereof in which ethylene glycol residues in bis(2-hydroxyethyl) terephthalate are substituted with other 1,4-butanediol residues. The recycled bis(4-hydroxybutyl) terephthalate comprises a component obtained by the depolymerization of waste polyester.

Therefore, the recycled bis(4-hydroxybutyl) terephthalate needs to be understood separately from a pure bis(4-hydroxybutyl) terephthalate compound. For this reason, recycled bis(4-hydroxybutyl) terephthalate can be viewed as a kind of composition comprising two or more components. That is, it can be understood as a bis(4-hydroxybutyl) terephthalate composition.

Specifically, the recycled bis(4-hydroxybutyl) terephthalate (recycled BHBT, abbreviated as r-BHBT or rBHBT) obtained by the transesterification reaction with the recycled BHET comprises a compound represented by the following Formula 1.

[Formula 1]

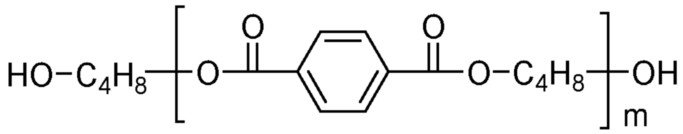

In Formula 1, m is an integer of 1 to 4.

That is, the recycled bis(4-hydroxybutyl) terephthalate may comprise a compound represented by Formula 1, that is, a monomer and two or more derivatives such as oligomers such as dimers and trimers.

However, the product of the transesterification reaction may comprise some unreacted materials or by-products in addition to a compound of Formula 1. Specifically, the product of the transesterification reaction may contain some impurities contained in recycled BHET as its raw material, reagents or solvents used in the transesterification reaction, or by-products formed by side reactions with them.

As an example, the product of the transesterification reaction may comprise unreacted 1,4-butanediol used as a starting material and a component derived from recycled bis(2-hydroxyethyl) terephthalate. Specifically, the product of the transesterification reaction may comprise ethylene glycol derivatives and diethylene glycol derivatives.

The ethylene glycol derivatives and diethylene glycol derivatives may be ethylene glycol or a compound having an ethylene glycol residue, for example, ethylene glycol, diethylene glycol, or an ester thereof. Specifically, the ethylene glycol derivative and diethylene glycol derivatives may comprise ethylene glycol, diethylene glycol, bis(2-hydroxyethyl) terephthalate, 2-hydroxyethyl terephthalate, 2-hydroxyethyl-4-hydroxybutyl terephthalate, bis(diethylene glycol) terephthalate, 2-hydroxydiethyl-diethylene glycol terephthalate, 4-hydroxybutyl-diethylene glycol terephthalate, and oligomers thereof.

As a specific example, the total content of ethylene glycol, diethylene glycol, and their derivatives present in the product of the transesterification reaction may be 20% by mole or less based on the total number of moles of the entire glycols and their derivatives. As a more specific example, the total content of ethylene glycol, diethylene glycol, and their derivatives may be 15% by mole or less based on the total number of moles of the entire glycols and their derivatives. The content is based on the amount of the entire glycols and their derivatives present in the product of the

transesterification reaction.

That is, the total content of ethylene glycol, diethylene glycol, and their derivatives in the recycled bis(4-hydroxybutyl) terephthalate according to the present invention as measured by $^1$H-NMR is 20% by mole or less based on the total number of moles of the entire glycols and their derivatives. For example, the total content of ethylene glycol, diethylene glycol, and their derivatives may be 15% by mole or less, 12% by mole or less, or 10% by mole or less. Meanwhile, the lower limit of the total content of ethylene glycol, diethylene glycol, and their derivatives is not particularly limited, but it may be, for example, 0% by mole or more, greater than 0% by mole, 0.01% by mole or more, 1% by mole or more, 3% by mole or more, or 5% by mole or more. The content of the ethylene glycol residues may be calculated as the total content of compounds containing ethylene glycol residues and diethylene glycol residues based on the amount of all compounds by obtaining and analyzing a $^1$H-NMR spectrum for recycled BHBT.

Thus, the recycled bis(4-hydroxybutyl) terephthalate of the present invention can be used as a raw material for preparing a highly crystalline 1,4-butanediol-derived polyester resin, and the crystallinity of the polyester resin may be enhanced within the above content range, resulting in more excellent thermal resistance and mechanical properties.

Recycled bis(4-hydroxybutyl) terephthalate prepared according to the present invention may be crushed or pulverized at room temperature. Specifically, the recycled bis(4-hydroxybutyl) terephthalate may be crushed or pulverized into flakes of 0.2 g or less at room temperature. Thus, the recycled bis(4-hydroxybutyl) terephthalate may be stored in a flake or powder state before being used as a polymerization raw material.

The recycled bis(4-hydroxybutyl) terephthalate according to the present invention has a color-b value of 8 or less in the Hunter Lab color space when prepared into a specimen having a thickness of 6 mm. Specifically, the color-b value may be 7 or less, 6 or less, 5 or less, 4 or less, or 3 or less. Meanwhile, the lower limit of the color-b value is not particularly limited, but it may be, for example, 0 or more, greater than 0, 1 or more, or 2 or more.

According to a specific embodiment, the total content of ethylene glycol, diethylene glycol, and their derivatives may be 15% by mole or less based on the total number of moles of the entire glycols and their derivatives, and the color-b value may be 5 or less.

According to the present invention, since the residual amount of ethylene glycol derivatives in recycled bis(4-hydroxybutyl) terephthalate is low, it is possible to prepare a 1,4-butanediol-derived polybutylene terephthalate-based polyester resin (e.g., PBT or TPEE) with high purity and to produce high-value engineering polyester products or biodegradable polyester (e.g. PBAT) products with a low content of ethylene glycol, which affects ecotoxicity, from waste PET products. In addition, since the product of recycled bis(4-hydroxybutyl) terephthalate according to the present invention is easily crushed to produce flakes or powder and has excellent color, it is suitable for use as a polymerization raw material.

According to another aspect of the present invention, there is provided a raw material for the polymerization of a polyester resin, which comprises the recycled bis(4-hydroxybutyl) terephthalate.

**Preparation of a polyester resin (polycondensation reaction)**

The product of the transesterification reaction (i.e., a mixture containing a recycled bis(4-hydroxybutyl) terephthalate) may be subjected to a polycondensation reaction to prepare a polyester resin.

The process for preparing a polyester resin according to an embodiment of the present invention comprises (1) feeding 1,4-butanediol to a reactor; (2) feeding bis(2-hydroxyethyl) terephthalate to the reactor in a divided or continuous manner and carrying out a transesterification reaction; and (3) subjecting the product of the transesterification reaction to a polycondensation reaction, wherein the components fed to the reactor satisfy the above Relationship (1).

The polycondensation reaction may be carried out, for example, by reacting the transesterification reaction product at a temperature of 150°C to 300°C and under a reduced pressure condition of 0.01 mmHg to 600 mmHg for 1 hour to 24 hours.

The temperature in the polycondensation reaction may be 150°C to 300°C, specifically, 200°C to 290°C, more specifically, 260°C to 280°C. In addition, the pressure in the polycondensation reaction may be 0.01 mmHg to 600 mmHg, specifically, 0.05 mmHg to 200 mmHg, more specifically, 0.1 mmHg to 100 mmHg. Within the temperature and pressure of the polycondensation reaction, a glycol, which is a by-product of the polycondensation reaction, can be advantageously removed from the system. In addition, the polycondensation reaction may be carried out for a necessary period of time, for example, an average residence time of 1 hour to 24 hours, until the intrinsic viscosity of a final reaction product reaches an appropriate level.

As the catalyst for the polycondensation reaction, for example, at least one selected from the group consisting of a titanium-based catalyst, a germanium-based catalyst, an antimony-based catalyst, an aluminum-based catalyst, and a tin-based catalyst may be used. Examples of the titanium-based catalyst include tetraethyl titanate, acetyltripropyl titanate, tetrapropyl titanate, tetrabutyl titanate, 2-ethylhexyl titanate, octylene glycol titanate, triethanolamine titanate, acetylacetonate titanate, ethylacetoacetic ester titanate, isostearyl titanate, titanium dioxide, and the like. Examples of the germanium-based catalyst include germanium dioxide, germanium tetrachloride, germanium ethylene glycol oxide,

germanium acetate, or combinations thereof. Specifically, germanium dioxide can be used as the germanium-based catalyst. Both crystalline and amorphous germanium dioxide may be used as the germanium dioxide, and glycol-soluble ones may also be used.

In addition, at least one of glycol components and acid components may be further introduced into the polycondensation reaction.

For example, specific types of the glycol components that may be further introduced into the polycondensation reaction may be a glycol monomer (e.g., alkylene glycol) or a polymer glycol (e.g., polyether). Specifically, the glycol component may be selected from the group consisting of 1,3-propanediol, 1,4-cyclohexanedimethanol, 2-methyl-1,3-propanediol, 2-methylene-1,3-propanediol, 2-ethyl-1,3-propanediol, 2-isopropyl-1,3-propanediol, 2,2-dimethyl-1,3-propanediol, 2,3-butanediol, 3-methyl-1,5-pentanediol, 3-methyl-2,4-pentanediol, 1,6-hexanediol, 1,2-cyclohexanediol, 1,4-cyclohexanediol, diethylene glycol, polyethylene glycol, polypropylene glycol, polytetramethylene glycol, polyhexamethylene glycol, copolymers of ethylene oxide and tetrahydrofuran, ethylene oxide-adducted polypropylene glycol, polycarbonate diol, polyneopentyl glycol, poly-3-methylpentanediol, and poly-1,5-pentanediol.

In addition, the acid component that may be further introduced into the polycondensation reaction may be, for example, at least one selected from dicarboxylic acids and esters thereof. Specifically, the acid component may be selected from the group consisting of adipic acid, sebacic acid, succinic acid, isodecylsuccinic acid, maleic acid, fumaric acid, glutaric acid, azelaic acid, 1,4-cyclohexanedicarboxylic acid, 1,3-cyclohexanedicarboxylic acid, terephthalic acid, isophthalic acid, naphthalenedicarboxylic acid, diphenyldicarboxylic acid, 4,4'-stilbendicarboxylic acid, 2,5-furandicarboxylic acid, and 2,5-thiophenedicarboxylic acid.

Alternatively, ethylene glycol may be introduced as an additional glycol component, and terephthalic acid may be introduced as an additional acid component, into the polycondensation reaction, if necessary.

**Polyester resin**

According to another aspect of the present invention, there is provided a polyester resin prepared by the above process. That is, since the polyester resin is prepared by the polycondensation of a recycled bis(4-hydroxybutyl) terephthalate obtained by a transesterification reaction, it comprises polymers of the recycled bis(4-hydroxybutyl) terephthalate. Therefore, the polyester resin comprises units derived from components derived from the recycled bis(4-hydroxybutyl) terephthalate.

In addition, since the recycled bis(4-hydroxybutyl) terephthalate comprises unreacted glycol and acid components or by-products such as ethylene glycol, in addition to bis(4-hydroxybutyl) terephthalate, units derived from these components may be contained in the polyester resin through the subsequent polycondensation.

Specifically, the polyester resin may comprise units derived from one or more glycol components; and units derived from one or more acid components. The one or more glycol components may be glycol monomers or polymer glycols exemplified above. The one or more acid components may be aliphatic dicarboxylic acids and aromatic dicarboxylic acids exemplified above.

The glycol component and acid component that constitute the polyester resin are those derived from bis(2-hydroxyethyl) terephthalate and the glycol components initially introduced for the preparation of the polyester resin, or a recycled bis(4-hydroxybutyl) terephthalate prepared therefrom, and acid components additionally introduced.

The polyester resin may be a homopolymer or copolymer resin. As an example, the polyester resin may be a homopolymer resin comprising 1,4-butanediol as a glycol component and terephthalic acid as an acid component, that is, a polybutylene terephthalate (PBT) resin. As another example, the polyester resin may be a copolymer resin comprising 1,4-butanediol and an additional glycol as a glycol component, and terephthalic acid and an additional acid as an acid component. The additional glycol and acid components may be the glycol and acid components previously exemplified as being able to be additionally introduced to the polycondensation step in the process for preparing a polyester resin.

The polyester resin may have a total content of ethylene glycol residues and diethylene glycol residues at a certain level or lower. For example, the total content of ethylene glycol residues and diethylene glycol residues of the polyester resin may be 20% by mole or less, 15% by mole or less, 10% by mole or less, 8% by mole or less, 7.5% by mole or less, 7% by mole or less, 6% by mole or less, 5% by mole or less, 4% by mole or less, or 3% by mole or less, based on the number of moles of the entire glycol residues. In addition, the total content of ethylene glycol residues and diethylene glycol residues may be 0% by mole or more, 0.01% by mole or more, 0.02% by mole or more, or 0.03% by mole or more. The total content of ethylene glycol residues and diethylene glycol residues may be calculated by analyzing a spectrum of the polyester resin obtained by $^1$H-NMR. These residues may be derived from ethylene glycol, diethylene glycol, and their derivatives. As a specific example, the polyester resin may have a total content of ethylene glycol residues and diethylene glycol residues of 0.01% by mole to 20% by mole based on the number of moles of the entire glycol residues when measured by $^1$H-NMR. As a more specific example, the total content of ethylene glycol residues and diethylene glycol residues may be 0.01% by mole to 15% by mole, or 0.1% by mole to 10% by mole, based on the number of moles of the entire glycol residues. Within the above content range, the crystallinity of the polyester resin

may be enhanced so that thermal resistance and mechanical properties may be more excellent.

The polyester resin may have an intrinsic viscosity of 0.5 dl/g to 1.5 dl/g at 35°C. For example, the intrinsic viscosity of the polyester resin at 35°C may be 0.5 dl/g or more, 0.55 dl/g or more, 0.6 dl/g or more, 0.7 dl/g or more, 0.8 dl/g or more, 0.9 dl/g or more, or 1.0 dl/g or more, and may be 1.5 dl/g or less, 1.4 dl/g or less, 1.3 dl/g or less, 1.2 dl/g or less, 1.1 dl/g or less, 1.0 dl/g or less, or 0.7 dl/g or less. As a specific example, the polyester resin may have an intrinsic viscosity of 0.55 to 1.1 dl/g or 0.6 to 0.7 dl/g at 35°C.

The polyester resin may have a melting point (Tm) of 100°C or higher, 150°C or higher, or 200°C or higher, and 300°C or lower, 280°C or lower, 260°C or lower, or 240°C or lower. The polyester resin may have a heat of fusion ($\Delta H\varepsilon$) of 10 J/g or more, 15 J/g or more, or 20 J/g or more, and 60 J/g or less, 50 J/g or less, or 40 J/g or less. As a specific example, the polyester resin may have a melting point (Tm) of 100°C or higher and a heat of fusion ($\Delta H\varepsilon$) of 10 J/g or more. As a more specific example, the polyester resin may have a melting point (Tm) of 200°C or higher and a heat of fusion ($\Delta H\varepsilon$) of 20 J/g or more.

The polyester resin according to the present invention has a color-b value of 18 or less in the Hunter Lab color space when prepared into a specimen having a thickness of 6 mm. Specifically, the color-b value may be 15 or less, 13 or less, 11 or less, 10 or less, or 9 or less. Meanwhile, the lower limit of the color-b value is not particularly limited, but it may be, for example, 0 or more, greater than 0, 1 or more, or 2 or more.

The polyester resin prepared by the above process may have a state such as chips, pellets, or powder before molding. It may also have a shape of a molded article formed by a separate molding process such as extrusion or injection, for example, a film or sheet form, or various injection part forms for automobiles, electrical and industrial purposes.

Accordingly, the present invention also provides an article, which comprises the polyester resin.

In particular, the polyester resin of the present invention can be utilized as high value-added engineering polyester products or biodegradable polyester products with low ethylene glycol components that affect ecotoxicity.

## Mode for the Invention

Hereinafter, a preferred embodiment is presented for the understanding of the present invention. However, the following examples are provided only to help easily understand the present invention, and the scope of the present invention is not limited thereby.

### <Preparation of recycled bis(2-hydroxyethyl) terephthalate>

### Preparation Example: rBHET1

A reactor made of stainless steel (SUS) was charged with 1,000 g of a waste polyester resin, 4,000 g of ethylene glycol, and 3.5 g of zinc acetate anhydride. The temperature inside the reactor was raised to 196°C, and depolymerization by a glycolysis reaction was carried out for 4 hours.

The reaction resultant was cooled to 30°C, and the crystallization of bis(2-hydroxyethyl) terephthalate was carried out for 2 hours. The slurry of bis(2-hydroxyethyl) terephthalate and ethylene glycol thus obtained was subjected to solid-liquid separation in a centrifugal separator.

Bis(2-hydroxyethyl) terephthalate obtained through centrifugation was washed twice with sufficient distilled water, and the residual solvent was removed in an oven to obtain about 1,100 g of a final product containing bis(2-hydroxyethyl) terephthalate.

### Preparation Examples: rBHET2 and rBHET3

Recycled bis(2-hydroxyethyl) terephthalates with various purities were prepared in a similar manner to the Preparation Example of rBHET1, except that the purification process was adjusted.

To confirm the purity of the recycled bis(2-hydroxyethyl) terephthalates thus prepared, 0.01 g of each sample (rBHET1, rBHET2, or rBHET3) was diluted in 20 mL of methanol or chloroform and subjected to high-performance liquid chromatography (HPLC). In the HPLC spectrum, the area of the BHET peak and the area of the impurities (other oligomers, DEG derivatives, and unreacted monomers) peaks were each integrated and calculated as a fraction (%) of the total peak area. The results are shown in the Table below.

[Table 1]

| | | rBHET1 | rBHET2 | rBHET3 |
|---|---|---|---|---|
| HPLC (%) | BHET | 97-98% | 75-80% | 60-65% |
| | Impurities | 2-3% | 20-25% | 35-40% |

**<Preparation of recycled bis(4-hydroxybutyl) terephthalate and a polyester resin>**

**Example 1**

Step (1): Preparation of recycled bis(4-hydroxybutyl) terephthalate through a transesterification reaction

A 1-liter reactor for a transesterification reaction equipped with a column and a condenser that can be cooled by water was charged with 614 g of 1,4-butanediol (BD) as a glycol component and 58 mg of tetrabutyl titanate (TBT) as a reaction catalyst. Then, the pressure in the reactor was adjusted to 0.1 kg/$cm^2$ by flowing nitrogen, and the temperature was raised while the pressure was maintained with stirring. When the temperature in the reactor reached about 190°C, while the temperature was maintained at 190°C over 4 hours, 96.2 g of bis(2-hydroxyethyl) terephthalate (rBHET2) was fed as divided into 6 portions to carry out a transesterification reaction. Glycols as by-products were discharged through the column and condenser during the reaction. Even upon completion of the addition of BHET, the transesterification reaction was continued while maintaining 190°C until the discharge of glycols stopped. Upon completion of the transesterification reaction, nitrogen in the pressurized reactor was released to the outside to lower the pressure in the reactor to normal pressure, and the resultant (a mixture containing bis(4-hydroxybutyl) terephthalate) in the reactor was then discharged to the outside to form a sheet. It was solidified by natural cooling and then crushed to obtain 700 g of recycled bis(4-hydroxybutyl) terephthalate in the form of flakes with an average weight of about 0.5 to 1 g.

Step (2): Preparation of a polyester resin through a polycondensation reaction

300.7 g of the recycled bis(4-hydroxybutyl) terephthalate (rBHBT) obtained in step (1) was fed to a polycondensation reactor. The pressure of the reactor was reduced from normal pressure to 5.0 Torr (absolute pressure: 5 mmHg) over 30 minutes. At the same time, the temperature of the reactor was raised to 245°C over 1 hour, and a polycondensation reaction was carried out while the pressure of the reactor was maintained at 1 Torr (absolute pressure: 1 mmHg) or less. At the beginning of the polycondensation reaction, the stirring speed might be set high. As the polycondensation reaction proceeded, the glycol component as a by-product was discharged from the reactor. When the stirring power was weakened due to the increase in the viscosity of the reactants or the temperature of the reactants was elevated above the set temperature, the stirring speed was appropriately adjusted. The polycondensation reaction was carried out until the intrinsic viscosity (IV) of the mixture (melt) in the reactor reached about 0.86 dl/g. When the intrinsic viscosity of the mixture in the reactor reached the desired level, the mixture was then discharged to the outside of the reactor to form strands. They were solidified with a cooling liquid and then granulated to have an average weight of about 12 to 14 mg to obtain 213.3 g of a polyester resin.

**Example 2**

The same procedure as in Example 1 was repeated to obtain 196.2 g of a polyester resin, except that, in step (1), 696 g of 1,4-butanediol (BD) was fed, and 577 g of recycled bis(2-hydroxyethyl) terephthalate (rBHET1) was dissolved in 3,861 g of purified water at 90°C to prepare an aqueous solution of 13% by weight, and the aqueous solution of bis(2-hydroxyethyl) terephthalate was continuously supplied at a rate of 18.5 g per minute for 4 hours using a dropping funnel; and that, in step (2), 199.2 g of recycled bis(4-hydroxybutyl) terephthalate (rBHBT) and 62.6 g of adipic acid (AA) were fed to a polycondensation reactor, and a polycondensation reaction was carried out until the intrinsic viscosity (IV) of the mixture (melt) in the reactor reached about 1.23 dl/g.

**Example 3**

The same procedure as in Example 1 was repeated to obtain 232.4 g of a polyester resin, except that, in step (1), 368 g of 1,4-butanediol (BD) was fed, 577 g of recycled bis(2-hydroxyethyl) terephthalate (rBHET1) was fed as divided into 12 portions, and the final temperature during the transesterification reaction was 220°C; and that, in step (2), 218.4 g of recycled bis(4-hydroxybutyl) terephthalate (rBHBT) and 77.4 g of polytetramethylene glycol (PTMG, Mn 1,000) were fed to a polycondensation reactor, and a polycondensation reaction was carried out until the intrinsic viscosity

(IV) of the mixture (melt) in the reactor reached about 1.35 dl/g.

**Example 4**

The same procedure as in Example 1 was repeated to obtain 234.3 g of a polyester resin, except that, in step (1), 577 g of recycled bis(2-hydroxyethyl) terephthalate (rBHET2) was fed as divided into 4 portions, and the final temperature during the transesterification reaction was 200°C; and that, in step (2), 220.2 g of recycled bis(4-hydroxybutyl) terephthalate (rBHBT) and 77.4 g of poly-1,3-propylene glycol (PO3G, Mn 1,000) were fed to a polycondensation reactor, and a polycondensation reaction was carried out until the intrinsic viscosity (IV) of the mixture (melt) in the reactor reached about 1.25 dl/g.

**Example 5**

The same procedure as in Example 1 was repeated to obtain 235.5 g of a polyester resin, except that, in step (1), 577 g of recycled bis(2-hydroxyethyl) terephthalate (rBHET1) was fed as divided into 8 portions, and the final temperature during the transesterification reaction was 215°C; and that, in step (2), 220.2 g of recycled bis(4-hydroxybutyl) terephthalate (rBHBT) and 83.1 g of ethylene oxide-adducted polypropylene glycol (EO-PPG, Mn 2,400) were fed to a polycondensation reactor, and a polycondensation reaction was carried out until the intrinsic viscosity (IV) of the mixture (melt) in the reactor reached about 1.23 dl/g.

**Example 6**

The same procedure as in Example 1 was repeated to obtain 233.9 g of a polyester resin, except that, in step (1), the amount of 1,4-butanediol (BD) fed was reduced to 491 g, 577 g of recycled bis(2-hydroxyethyl) terephthalate (rBHET1) was fed as divided into 10 portions, and the final temperature during the transesterification reaction was 170°C; and that, in step (2), 219.8 g of recycled bis(4-hydroxybutyl) terephthalate (rBHBT) and 78.0 g of polyethylene glycol (PEG, Mn 1,000) were fed to a polycondensation reactor, and a polycondensation reaction was carried out until the intrinsic viscosity (IV) of the mixture (melt) in the reactor reached about 1.29 dl/g.

**Comparative Example 1**

The same procedure as in Example 1 was repeated to obtain 216.0 g of a polyester resin, except that, in step (1), recycled bis(2-hydroxyethyl) terephthalate (rBHET3) was fed together with 1,4-butanediol (BD) at one time at the beginning; and that, in step (2), 304.4 g of recycled bis(4-hydroxybutyl) terephthalate (rBHBT) was fed to a polycondensation reactor, and a polycondensation reaction was carried out until the intrinsic viscosity (IV) of the mixture (melt) in the reactor reached about 0.62 dl/g.

**Comparative Example 2**

The same procedure as in Comparative Example 1 was repeated to obtain 187.7 g of a polyester resin, except that, in step (1), the amount of 1,4-butanediol (BD) fed was reduced to 307 g, and recycled bis(2-hydroxyethyl) terephthalate (rBHET2) was fed together therewith at one time at the beginning; and that, in step (2), 190.5 g of recycled bis(4-hydroxybutyl) terephthalate (rBHBT) and 59.8 g of adipic acid (AA) were fed to a polycondensation reactor, and a polycondensation reaction was carried out until the intrinsic viscosity (IV) of the mixture (melt) in the reactor reached about 0.94 dl/g.

**Comparative Example 3**

The same procedure as in Comparative Example 1 was repeated to obtain 246.4 g of a polyester resin, except that, in step (1), recycled bis(2-hydroxyethyl) terephthalate (rBHET2) was fed together with 1,4-butanediol (BD) at one time at the beginning, and the final temperature during the transesterification reaction was 155°C; and that, in step (2), 231.6 g of recycled bis(4-hydroxybutyl) terephthalate (rBHBT) and 82.1 g of polytetramethylene glycol (PTMG, Mn 1,000) were fed to a polycondensation reactor, and a polycondensation reaction was carried out until the intrinsic viscosity (IV) of the mixture (melt) in the reactor reached about 0.88 dl/g.

**Comparative Example 4**

The same procedure as in Comparative Example 1 was repeated to obtain 243.9 g of a polyester resin, except

that, in step (1), the amount of 1,4-butanediol (BD) fed was reduced to 409 g, recycled bis(2-hydroxyethyl) terephthalate (rBHET1) was fed together therewith at one time at the beginning, and the final temperature during the transesterification reaction was 230°C; and that, in step (2), 229.2 g of recycled bis(4-hydroxybutyl) terephthalate (rBHBT) and 81.3 g of poly-1,3-propylene glycol (PO3G, Mn 1,000) were fed to a polycondensation reactor, and a polycondensation reaction was carried out until the intrinsic viscosity (IV) of the mixture (melt) in the reactor reached about 1.08 dl/g.

**Comparative Example 5**

The same procedure as in Comparative Example 1 was repeated to obtain 243.9 g of a polyester resin, except that, in step (1), the amount of 1,4-butanediol (BD) fed was reduced to 409 g, recycled bis(2-hydroxyethyl) terephthalate (rBHET2) was fed together therewith at one time at the beginning, and the final temperature during the transesterification reaction was 210°C; and that, in step (2), 229.2 g of recycled bis(4-hydroxybutyl) terephthalate (rBHBT) and 81.3 g of polyethylene glycol (PEG, Mn 1,000) were fed to a polycondensation reactor, and a polycondensation reaction was carried out until the intrinsic viscosity (IV) of the mixture (melt) in the reactor reached about 1.08 dl/g.

**Comparative Example 6**

The same procedure as in Comparative Example 1 was repeated to obtain 218.0 g of a polyester resin, except that, in step (1), recycled bis(2-hydroxyethyl) terephthalate (rBHET3) was fed together with 1,4-butanediol (BD) at one time at the beginning, and recycled bis(4-hydroxybutyl) terephthalate was obtained without a nitrogen atmosphere; and that, in step (2), 307.2 g of recycled bis(4-hydroxybutyl) terephthalate (rBHBT) was fed to a polycondensation reactor, and a polycondensation reaction was carried out until the intrinsic viscosity (IV) of the mixture (melt) in the reactor reached about 0.95 dl/g.

**Comparative Example 7**

The same procedure as in Comparative Example 1 was repeated to obtain 215.4 g of a polyester resin, except that, in step (1), the amount of 1,4-butanediol (BD) fed was increased to 1,023 g, recycled bis(2-hydroxyethyl) terephthalate (rBHET2) was fed together with 1,4-butanediol (BD) at one time at the beginning, and recycled bis(4-hydroxybutyl) terephthalate was obtained without a nitrogen atmosphere; and that, in step (2), 303.6 g of recycled bis(4-hydroxybutyl) terephthalate (rBHBT) was fed to a polycondensation reactor, and a polycondensation reaction was carried out until the intrinsic viscosity (IV) of the mixture (melt) in the reactor reached about 0.95 dl/g.

The components used in the above Examples and Comparative Examples, their amounts employed, and other detailed process conditions are shown in Tables 2 and 3 below.

In addition, the 1,4-butanediol/rBHET ratio (G/B) of the following Relationship (1) and the rBHET introduction ratio (R) of the following Relationship (2) were calculated and shown in Tables 2 and 3 below.

$$1.5 \leq G/B \leq 3.5 \; ... \; (1)$$

In Relationship (1), G is the total number of moles of 1,4-butanediol, and B is the total number of moles of the bis(2-hydroxyethyl) terephthalate.

$$R = G \times \frac{N}{B} \quad ... \; (2)$$

In Relationship (2), G is the total number of moles of 1,4-butanediol, B is the total number of moles of the bis(2-hydroxyethyl) terephthalate, and N is the number of divided introductions of the bis(2-hydroxyethyl) terephthalate.

**Test Example 1: Crushability**

The recycled bis(4-hydroxybutyl) terephthalate sheets discharged from the transesterification reaction in the Examples and Comparative Examples were each struck with a rubber hammer or broken by a press with a light load into chunks equivalent to 50 g or more. If they were crushed into flakes of 0.2 g or less by a ball mill or pin mill at room temperature, they were evaluated as "brittle." If they were not crushed (i.e., tacky or waxy), they were evaluated as "waxy."

**Test Example 2: Color-b**

The recycled bis(4-hydroxybutyl) terephthalates obtained in the Examples and Comparative examples were each prepared into a specimen with a width of 30 mm, a length of 30 mm, and a thickness of 6 mm through hot press melting or injection. Transmission data was obtained with Illuminant D65 at an observer angle of 2°, and the data was processed using a color analysis device in Grams/32 software to measure the color-b value of the Hunter Lab color space. In addition, the polyester resins obtained in the Examples and Comparative Examples were each prepared into a specimen with a width of 30 mm, a length of 30 mm, and a thickness of 6 mm through hot press melting or injection in the same manner as above, and the color-b value was measured in the same manner as above.

**Test Example 3: $^1$H-NMR**

(1) Analysis of recycled bis(4-hydroxybutyl) terephthalate

The recycled bis(4-hydroxybutyl) terephthalates obtained in the Examples and Comparative examples were each dissolved in a $CDCl_3$ solvent at a concentration of 3 mg/ml, whose $^1$H-NMR spectrum was obtained at 25°C using nuclear magnetic resonance equipment (JEOL, 600MHz FT-NMR). The spectrum was analyzed to calculate the total content of bis(4-hydroxybutyl) terephthalate (BHBT) and its oligomers, which was used as the content of BHBT derivatives (% by mole). In addition, the total content (% by mole) of ethylene glycol (EG), diethylene glycol (DEG), and their derivatives was calculated based on the total number of moles of the entire glycols (EG, DEG, BD, and the like) and their derivatives. In addition, the content (% by mole) of unreacted 1,4-butanediol based on the total number of moles of the entire glycols and their derivatives was calculated. The content was based on the amount of all compounds analyzed through a $^1$H-NMR spectrum.

(2) Analysis of a polyester resin

Each polyester resin was dissolved in a $CDCl_3$ solvent at a concentration of 3 mg/ml, whose $^1$H-NMR spectrum was obtained at 25°C using nuclear magnetic resonance equipment (JEOL, 600MHz FT-NMR). The total content (% by mole) of ethylene glycol residues and diethylene glycol residues based on the total number of moles of residues derived from the entire glycols (EG, DEG, BD, PEG, PTMG, PO3G, EO-PPG, and the like) was calculated by analyzing the above spectra.

**Test Example 4: Intrinsic viscosity**

A polyester resin was dissolved at a concentration of 0.12% in orthochlorophenol (OCP) at 150°C to obtain a solution, and an Ubbelohde viscometer was used in a constant temperature bath at 35°C to measure intrinsic viscosity. Specifically, the temperature of the viscosity tube was maintained at 35°C, and the time (efflux time) required for the solvent to pass between specific internal sections of the viscosity tube and the time required for the solution to pass to obtain specific viscosity, which was used to calculate intrinsic viscosity.

**Test Example 5: Tm and ∆Hf**

Each polyester resin was dried under a reduced pressure at 50°C for 15 hours, melted, and quenched, which was then heated at 10°C/minute in a differential scanning calorimeter (DSC, TA Instruments) for testing. The highest point of the endothermic peak by resin melting was taken as the melting point (Tm), and the heat of fusion (∆Hε) was calculated as the area of the endothermic peak.

The raw materials employed, process conditions, and test results for the preparation of recycled bis(4-hydroxybutyl) terephthalate through a transesterification reaction are shown in Tables 2 and 3 below.

[Table 2]

| | | Ex. 1 | Ex. 2 | Ex. 3 | Ex. 4 | Ex. 5 | Ex. 6 |
|---|---|---|---|---|---|---|---|
| Raw material feeding | rBHET1 (g) | - | 577 | 577 | - | 577 | 577 |
| | rBHET2 (g) | 577 | - | - | 577 | - | - |
| | rBHET3 (g) | - | - | - | - | - | - |
| | BD (g) | 614 | 696 | 368 | 614 | 614 | 491 |
| | Catalyst (mg) | 58 | 58 | 58 | 29 | 5.8 | 144 |
| | G/B | 3.0 | 3.4 | 1.8 | 3.0 | 3.0 | 2.4 |
| Process Condition | Number of divided feedings of rBHET | 6 | Continuous feeding | 12 | 4 | 8 | 10 |
| | R | 18 | - | 21.6 | 12 | 24 | 24 |
| | Reaction temp. (°C) | 190 | 190 | 220 | 200 | 215 | 170 |
| | $N_2$ blow | ○ | ○ | ○ | ○ | ○ | ○ |
| | Reaction time (h) | 5 | 5.5 | 4 | 6.5 | 7 | 7 |
| Evaluation | Crushability | brittle | brittle | brittle | brittle | brittle | brittle |
| | rBHBT Color-b | 2.8 | 2.7 | 4.8 | 3.9 | 4.5 | 4.7 |
| NMR (% by mole) | Unreacted BD | 0.4 | 0.4 | 0.2 | 0.3 | 0.2 | 0.5 |
| | EG/DEG derivatives | 10.1 | 3.0 | 9.5 | 14.0 | 13.5 | 13.1 |
| | BHBT oligomer | 18.2 | 16.4 | 29.4 | 22.3 | 13.9 | 8.1 |
| | BHBT | 71.3 | 80.2 | 60.9 | 63.4 | 72.4 | 78.3 |
| | BHBT derivatives | 89.5 | 96.6 | 90.3 | 85.7 | 86.3 | 86.4 |

[Table 3]

| | | C. Ex. 1 | C. Ex. 2 | C. Ex. 3 | C. Ex. 4 | C. Ex. 5 | C. Ex. 6 | C. Ex. 7 |
|---|---|---|---|---|---|---|---|---|
| Raw material feeding | rBHET1 (g) | - | - | - | 577 | - | - | - |
| | rBHET2 (g) | - | 577 | 577 | | 577 | - | 577 |
| | rBHET3 (g) | 577 | - | - | - | - | 577 | - |
| | BD (g) | 614 | 307 | 614 | 409 | 409 | 614 | 1,023 |
| | Catalyst (mg) | 58 | 58 | 58 | 231 | 289 | 58 | 58 |
| | G/B | 3 | 1.5 | 3 | 2 | 2 | 3 | 4.5 |
| Process Condition | Number of divided feedings of rBHET | 1 | 1 | 1 | 1 | 1 | 1 | 1 |
| | R | 3 | 1.5 | 3 | 2 | 2 | 3 | 4.5 |
| | Reaction temp. (°C) | 190 | 190 | 155 | 230 | 210 | 190 | 190 |
| | $N_2$ blow | ○ | ○ | ○ | ○ | ○ | × | × |
| | Reaction time (h) | 4 | 5.5 | 11 | 4 | 4 | 9 | 9 |
| Evaluation | Crushability | brittle | brittle | waxy | brittle | brittle | waxy | waxy |
| | rBHBT Color-b | 8.3 | 8.4 | 10.5 | 9.1 | 13.2 | 8.8 | 9.1 |

(continued)

| | | C. Ex. 1 | C. Ex. 2 | C. Ex. 3 | C. Ex. 4 | C. Ex. 5 | C. Ex. 6 | C. Ex. 7 |
|---|---|---|---|---|---|---|---|---|
| NMR (% by mole) | Unreacted BD | 0.4 | 0.4 | 4.6 | 0.1 | 0.2 | 3.5 | 6.8 |
| | EG/DEG derivatives | 43.8 | 25.5 | 44.9 | 22.7 | 23.9 | 43.6 | 23.7 |
| | BHBT oligomer | 34.3 | 67.4 | 12.3 | 70.3 | 71.1 | 24.8 | 24.2 |
| | BHBT | 21.5 | 6.7 | 38.2 | 6.9 | 4.8 | 28.1 | 45.3 |
| | Total BHBT derivatives | 55.8 | 74.1 | 50.5 | 77.2 | 75.9 | 52.9 | 69.5 |

As can be seen from Tables 2 and 3 above, the transesterification reaction was carried out in a nitrogen atmosphere while recycled BHET was fed continuously or as divided at an introduction ratio (R) of 5 or more in Examples 1 to 6, whereby ethylene glycol (EG) as a reaction by-product and unreacted 1,4-butanediol (BD) could be effectively removed. As a result, in Examples 1 to 6, the content of ethylene glycol (EG) derivatives in the final product (recycled BHBT) was low at 20% by weight or lower, and the purity was excellent. The final product was readily crushed to be prepared into flakes or powder and had excellent color, making it suitable for use as a polymerization raw material.

In contrast, in Comparative Examples 1 to 7 in which the transesterification reaction was carried out while rBHET was fed at one time, the content of ethylene glycol (EG) derivatives in the final product (recycled BHBT) was high, exceeding 20% by mole. In particular, it was difficult to crush the final product in Comparative Examples 3, 6, and 7, and the color was poor in Comparative Examples 3 to 5, whereby they were not suitable as polymerization raw materials.

In addition, the feeding amount of raw materials, process conditions, and test results for the preparation of a polyester resin through a polycondensation reaction are shown in Tables 4 and 5 below.

[Table 4]

| | | Ex. 1 | Ex. 2 | Ex. 3 | Ex. 4 | Ex. 5 | Ex. 6 |
|---|---|---|---|---|---|---|---|
| Raw material | rBHBT (g) | 300.7 | 199.2 | 218.4 | 220.2 | 220.2 | 219.8 |
| | AA (g) | - | 62.6 | - | - | - | - |
| | PTMG (g) | - | - | 77.4 | - | - | - |
| | PO3G (g) | - | - | - | 77.4 | - | - |
| | PEG (g) | - | - | - | - | - | 78.0 |
| | EO-PPG (g) | - | - | - | - | 83.1 | - |
| Process Condition | Feeding amount (g) | 300.7 | 261.8 | 295.8 | 297.6 | 303.2 | 297.8 |
| | Yield (g) | 213.3 | 196.2 | 232.4 | 234.3 | 235.5 | 233.9 |
| | IV (dl/g) | 0.86 | 1.23 | 1.35 | 1.25 | 1.23 | 1.29 |
| Evaluation | EG/DE residues (% by mole) | 2.5 | 1.2 | 3.8 | 4.2 | 5.4 | 5.2 |
| | Color b of the final resin | 8.3 | 12.4 | 14.2 | 15.4 | 17.5 | 11.8 |
| | Tm (°C) | 219.4 | 121.3 | 202.1 | 203.9 | 214.5 | 200.3 |
| | $\Delta H_f$ (J/g) | 51.3 | 13.4 | 24.9 | 26.2 | 31.8 | 35.9 |

[Table 5]

| | | C. Ex. 1 | C. Ex. 2 | C. Ex. 3 | C. Ex. 4 | C. Ex. 5 | C. Ex. 6 | C. Ex. 7 |
|---|---|---|---|---|---|---|---|---|
| Raw material | rBHBT (g) | 304.4 | 190.5 | 231.6 | 229.2 | 229.2 | 307.2 | 303.6 |
| | AA (g) | - | 59.8 | - | - | - | - | - |
| | PTMG (g) | - | - | 82.1 | - | - | - | - |
| | PO3G (g) | - | - | - | 81.3 | - | - | - |
| | PEG (g) | - | - | - | - | 81.3 | - | - |
| | EO-PPG (g) | - | - | - | - | - | - | - |
| Process Condition | Feeding amount (g) | 304.4 | 250.4 | 313.7 | 310.4 | 310.5 | 307.2 | 303.6 |
| | Yield (g) | 216.0 | 187.7 | 246.4 | 243.9 | 243.9 | 218.0 | 215.4 |
| | IV (dl/g) | 0.62 | 0.94 | 0.88 | 1.12 | 1.08 | 0.95 | 0.95 |
| Evaluation | EG/DE residues (% by mole) | 26.3 | 20.4 | 35.9 | 21.1 | 21.5 | 26.2 | 22.5 |
| | Color b of the final resin | 19.4 | 23.4 | 22.4 | 21.5 | 24.9 | 20.8 | 22.3 |
| | Tm (°C) | 205.9 | 107.8 | 172.3 | 187.5 | 186.2 | 204.7 | 214.5 |
| | $\Delta H_f$ (J/g) | 34.3 | 2.2 | 7.6 | 16.4 | 17.3 | 32.4 | 44.3 |

As can be seen from Tables 4 and 5 above, a recycled BHBT with high quality prepared by the process (the transesterification reaction was carried out in a nitrogen atmosphere while recycled BHET was fed continuously or as divided at an introduction ratio (R) of 5 or more) according to the present invention was used in Examples 1 to 6; thus, the content of ethylene glycol (EG) residue in the final polyester resin was low at 10% by mole or lower.

In contrast, in Comparative Examples 1 to 7 in which a recycled BHBT with low quality was used, which had been prepared by the transesterification reaction carried out while recycled BHET was fed at one time, the content of ethylene glycol (EG) residue in the final polyester resin was high, exceeding 20% by mole.

As a result, the polyester resins of the Examples had a melting point ($T_m$) and a heat of fusion ($\Delta H\varepsilon$) higher than those of the polyester resins of the Comparative Examples, indicating that they had excellent crystallinity.

## Claims

1. Recycled bis(4-hydroxybutyl) terephthalate in which the total content of ethylene glycol, diethylene glycol, and their derivatives, as measured by $^1$H-NMR, is 20% by mole or less based on the total number of moles of the entire glycols and their derivatives, and, when it is prepared into a specimen having a thickness of 6 mm, the color-b value in the Hunter Lab color space is 8 or less.

2. The recycled bis(4-hydroxybutyl) terephthalate of claim 1, wherein the recycled bis(4-hydroxybutyl) terephthalate comprises a component obtained by the depolymerization of waste polyester, the total content of ethylene glycol, diethylene glycol, and their derivatives is 15% by mole or less based on the total number of moles of the entire glycols and their derivatives, and the color-b value is 5 or less.

3. The recycled bis(4-hydroxybutyl) terephthalate of claim 1, wherein the recycled bis(4-hydroxybutyl) terephthalate is capable of being crushed or pulverized into flakes of 0.2 g or less at room temperature.

4. A process for preparing recycled bis(4-hydroxybutyl) terephthalate, which comprises (1) feeding 1,4-butanediol to a reactor; and (2) feeding bis(2-hydroxyethyl) terephthalate to the reactor in a divided or continuous manner and carrying out a transesterification reaction,

   wherein the following Relationship (1) is satisfied:

$$1.5 \leq G/B \leq 3.5 \ldots (1)$$

in Relationship (1), G is the total number of moles of 1,4-butanediol fed to the reactor, and B is the total number of moles of bis(2-hydroxyethyl) terephthalate fed to the reactor.

**5.** The process for preparing recycled bis(4-hydroxybutyl) terephthalate of claim 4, wherein the purity of the bis(2-hydroxyethyl) terephthalate is 70% to 99%.

**6.** The process for preparing recycled bis(4-hydroxybutyl) terephthalate of claim 4, wherein the bis(2-hydroxyethyl) terephthalate is obtained by the depolymerization of waste polyester.

**7.** The process for preparing recycled bis(4-hydroxybutyl) terephthalate of claim 4, wherein the bis(2-hydroxyethyl) terephthalate is introduced into the transesterification reaction in step (2) in a divided manner of two or more times, and the introduction ratio (R) of bis(2-hydroxyethyl) terephthalate according to the following Relationship (2) is 5 or more:

$$R = \mathrm{G} \times \frac{\mathrm{N}}{B} \quad \ldots (2)$$

in Relationship (2), G is the total number of moles of the at least one glycol component, B is the total number of moles of the bis(2-hydroxyethyl) terephthalate, and N is the number of divided introductions of the bis(2-hydroxyethyl) terephthalate.

**8.** The process for preparing recycled bis(4-hydroxybutyl) terephthalate of claim 4, wherein, in step (2), the feeding of bis(2-hydroxyethyl) terephthalate is carried out in a nitrogen atmosphere at a temperature of 165°C to 220°C while ethylene glycol as a by-product is removed.

**9.** The process for preparing recycled bis(4-hydroxybutyl) terephthalate of claim 4, wherein, in step (2), the total content of ethylene glycol, diethylene glycol, and their derivatives present in the product of the transesterification reaction is 20% by mole or less based on the total number of moles of the entire glycols and their derivatives.

**10.** A raw material for the polymerization of a polyester resin, which comprises the recycled bis(4-hydroxybutyl) terephthalate of claim 1.

**11.** A process for preparing a polyester resin, which comprises:

(1) feeding 1,4-butanediol to a reactor;
(2) feeding bis(2-hydroxyethyl) terephthalate to the reactor in a divided or continuous manner and carrying out a transesterification reaction; and
(3) subjecting the product of the transesterification reaction to a polycondensation reaction, wherein the following Relationship (1) is satisfied:

$$1.5 \leq \mathrm{G/B} \leq 3.5 \ldots (1)$$

in Relationship (1), G is the total number of moles of 1,4-butanediol fed to the reactor, and B is the total number of moles of bis(2-hydroxyethyl) terephthalate fed to the reactor.

**12.** The process for preparing a polyester resin of claim 11, wherein at least one of glycol components and acid components is further fed in step (3),

the glycol component is selected from the group consisting of 1,3-propanediol, 1,4-cyclohexanedimethanol, 2-methyl-1,3-propanediol, 2-methylene-1,3-propanediol, 2-ethyl-1,3-propanediol, 2-isopropyl-1,3-propanediol, 2,2-dimethyl-1,3-propanediol, 2,3-butanediol, 3-methyl-1,5-pentanediol, 3-methyl-2,4-pentanediol, 1,6-hexanediol, 1,2-cyclohexanediol, 1,4-cyclohexanediol, diethylene glycol, polyethylene glycol, polypropylene glycol, polytetramethylene glycol, polyhexamethylene glycol, copolymers of ethylene oxide and tetrahydrofuran, ethylene oxide-adducted polypropylene glycol, polycarbonate diol, polyneopentyl glycol, poly-3-methylpentanediol, and poly-1,5-pentanediol; and

the acid component is selected from the group consisting of adipic acid, sebacic acid, succinic acid, isodecylsuccinic acid, maleic acid, fumaric acid, glutaric acid, azelaic acid, 1,4-cyclohexanedicarboxylic acid, 1,3-cyclohexanedicarboxylic acid, terephthalic acid, isophthalic acid, naphthalenedicarboxylic acid, diphenyldicarboxylic acid, 4,4'-stilbendicarboxylic acid, 2,5-furandicarboxylic acid, and 2,5-thiophenedicarboxylic acid.

**13.** A polyester resin, which is prepared by the process according to claim 11 or 12.

**14.** The polyester resin of claim 13, which has a total content of ethylene glycol residues and diethylene glycol residues of 0.01% by mole to 20% by mole based on the number of moles of the entire glycol residues when measured by $^{1}H$-NMR.

**15.** The polyester resin of claim 13, wherein the polyester resin has a melting point (Tm) of 200°C or higher, a heat of fusion ($\Delta H\varepsilon$) of 20 J/g or more, and a color-b value of 18 or less in the Hunter Lab color space when prepared into a specimen having a thickness of 6 mm.

[Fig. 1]

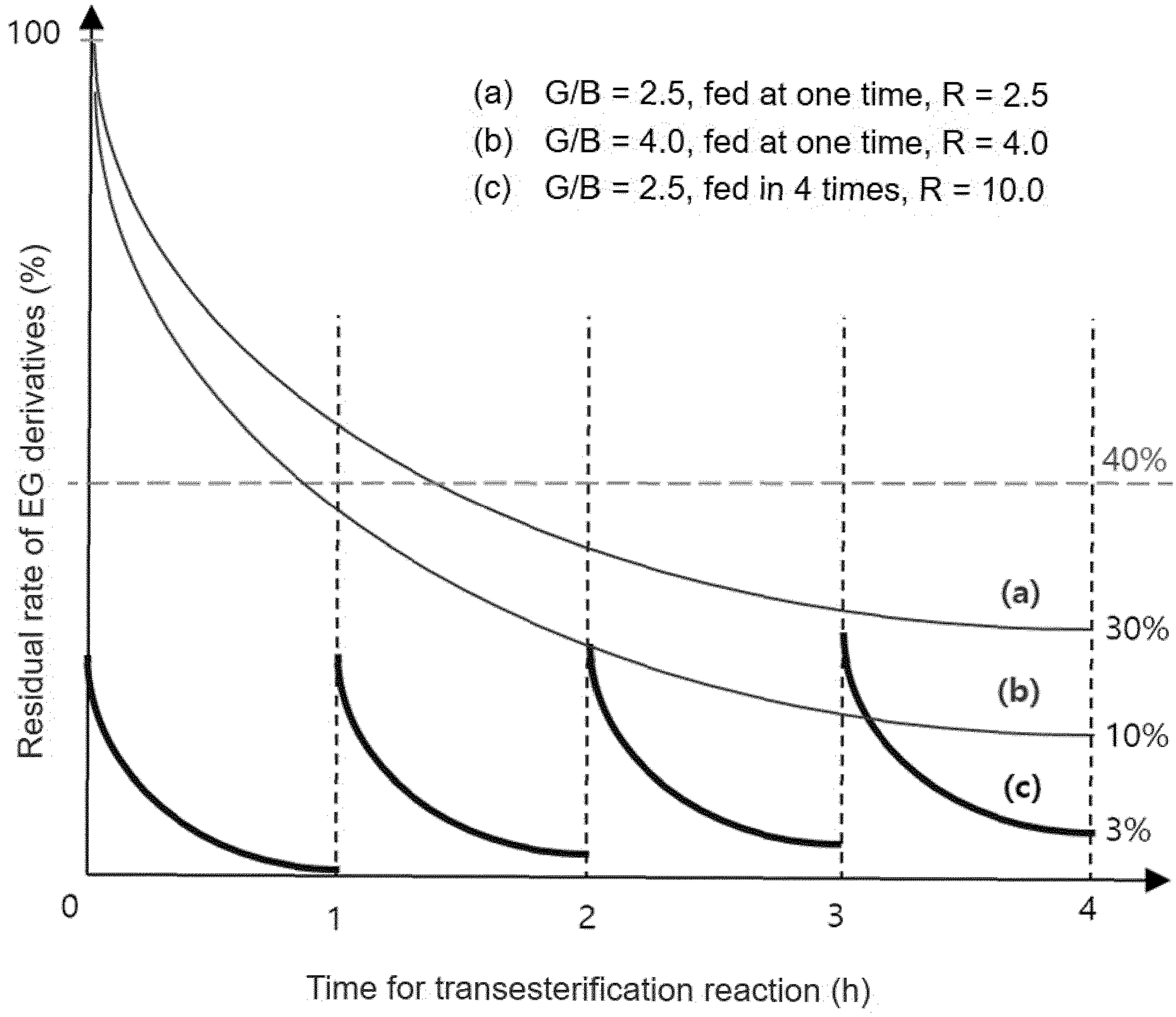
100
(a) G/B = 2.5, fed at one time, R = 2.5
(b) G/B = 4.0, fed at one time, R = 4.0
(c) G/B = 2.5, fed in 4 times, R = 10.0
Residual rate of EG derivatives (%)
40%
(a)
30%
(b)
10%
(c)
3%
0
1
2
3
4
Time for transesterification reaction (h)

**INTERNATIONAL SEARCH REPORT**

International application No.
**PCT/KR2023/018887**

**A. CLASSIFICATION OF SUBJECT MATTER**

**C07C 69/86**(2006.01)i; **C07C 67/60**(2006.01)i; **C07C 67/02**(2006.01)i; **C07C 67/03**(2006.01)i; **C08J 11/10**(2006.01)i; **C08G 63/60**(2006.01)i

According to International Patent Classification (IPC) or to both national classification and IPC

**B. FIELDS SEARCHED**

Minimum documentation searched (classification system followed by classification symbols)

C07C 69/86(2006.01); C07C 67/02(2006.01); C07C 67/03(2006.01); C07C 67/08(2006.01); C07C 69/675(2006.01); C08G 63/183(2006.01); C08G 63/78(2006.01); C08G 63/85(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, Caplus), Google & keywords: 재생 비스(4-히드록시부틸)테레프탈레이트 (recycled bis(4-hydroxybutyl)terephthalate, rBHET), 분할 투입(split input), 연속 투입(continuous input), 에틸렌글리콜(EG, ethylene glycol), 1,4-부탄디올(1,4-butanediol, BD), 순도(purity)

**C. DOCUMENTS CONSIDERED TO BE RELEVANT**

| Category* | Citation of document, with indication, where appropriate, of the relevant passages | Relevant to claim No. |
|---|---|---|
| Y | 전형철 등. Bis(2-Hydroxyethyl) Terephthalate와 1,4-Butanediol의 에스테르 교환 반응. Korean Chemical Engineering Research. 2018, vol. 56, no. 1, pp. 103-111 (JEON, Hyeongcheol et al. Transesterification Kinetics of Bis(2-Hydroxyethyl) Terephthalate with 1,4-Butandiol.)<br>See abstract; pages 103-105; and figures 4(a) and 4(b). | 1-15 |
| Y | KR 10-2125452 B1 (LG CHEM, LTD.) 22 June 2020 (2020-06-22)<br>See claim 1; and paragraphs [0018], [0031] and [0057]. | 1-15 |
| A | JP 2006-083124 A (TEIJIN LTD.) 30 March 2006 (2006-03-30)<br>See entire document. | 1-15 |
| A | KR 10-2001-0083551 A (SAEHAN INDUSTRIES INCORPORATION) 01 September 2001 (2001-09-01)<br>See entire document. | 1-15 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **04 March 2024** | **04 March 2024** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2022)

**INTERNATIONAL SEARCH REPORT**

International application No.
**PCT/KR2023/018887**

**C. DOCUMENTS CONSIDERED TO BE RELEVANT**

| Category* | Citation of document, with indication, where appropriate, of the relevant passages | Relevant to claim No. |
|---|---|---|
| PX | KR 10-2023-0143522 A (SK CHEMICALS CO., LTD.) 12 October 2023 (2023-10-12)<br>See paragraphs [0137]-[0143], [0179]-[0183], [0185], [0187] and [0193], and table 1. | 1-15 |

Form PCT/ISA/210 (second sheet) (July 2022)

**INTERNATIONAL SEARCH REPORT**
**Information on patent family members**

International application No.
**PCT/KR2023/018887**

| Patent document cited in search report | Publication date (day/month/year) | Patent family member(s) | Publication date (day/month/year) |
|---|---|---|---|
| KR 10-2125452 B1 | 22 June 2020 | KR 10-2018-0024320 A | 08 March 2018 |
| JP 2006-083124 A | 30 March 2006 | JP 4647271 B2 | 09 March 2011 |
| KR 10-2001-0083551 A | 01 September 2001 | KR 10-0603346 B1 | 20 July 2006 |
| KR 10-2023-0143522 A | 12 October 2023 | WO 2023-195668 A1 | 12 October 2023 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **PARK, S.H. ; KIM, S.H.** Polyethylene terephthalate) recycling for high value added textiles. *Fashion and Textiles,* 2014, vol. 1, 1